# EUROPÄISCHE PATENTSCHRIFT

(11) **EP 0 357 546 B1**
(45) Veröffentlichungstag und Bekanntmachung des Hinweises auf die Patenterteilung: **28.04.1993**
(21) Anmeldenummer: 89810529.1
(22) Anmeldetag: 13.07.1989
(51) Int. Cl.: A61F 2/30

(54) **Verankerungsschaft für eine Endoprothese**
Anchorage shank for an endoprosthesis
Tige d'ancrage pour endoprothèse

(30) Priorität: 23.08.1988 CH 3128/88
(43) Veröffentlichungstag der Anmeldung: 07.03.1990
(73) Patentinhaber: GEBRÜDER SULZER AKTIENGESELLSCHAFT, CH-8401 Winterthur (CH)
(72) Erfinder: Frey, Otto, Dr., CH-8400 Wintherthur (CH); Koch, Rudolf, CH-8267 Berlingen (CH)

(56) Entgegenhaltungen:
- EP-A- 0 178 650
- US-A- 4 064 567
- US-A- 4 718 909

## Beschreibung

Die Erfindung betrifft einen Verankerungsschaft zur Verankerung einer Endoprothese in einem Röhrenknochen mit Hilfe eines Knochenzementbettes, wobei der Verankerungsschaft zur Verankerung einer Endoprothese in einem Röhrenknochen mit Hilfe eines Knochenzementbettes, wobei der Schaft durch mindestens ein auf ihn aufgeschobenes, elastisch federndes Element relativ zur Wand des Operationshohlraumes in einer vorgegebenen Lage gehalten ist.

Bei - beispielsweise aus der US-A-4,064,567 oder der DE-A-22 46 940 - bekannten Verankerungsschäften dient ein federndes Element dazu, für das distale Ende des Schaftes in einem Knochenzementbett eine bestimmte Lage relativ zur Wand des operativ in dem Röhrenknochen geschaffenen Hohlraumes zu gewährleisten, in dem der Schaft verankert wird.

Aus der US-A-4,718,909 ist ein Verankerungsschaft bekannt, der etwa in der Mitte seiner axialen Länge durch einen elastisch federnden Distanzhalter im Abstand von der Wand des Operationshohlraumes gehalten ist. Dieser Distanzhalter ist bei einer der dortigen Ausführungsformen als spulenartig gewickelter in Umfangsrichtung geschlossener Ring ausgebildet. Die "Spulenwicklung" soll dabei so weitmaschig sein, dass der Ring für den nachträglich eingebrachten Knochenzement durchlässig ist. Bei neuen Zementiertechniken wird der Schaft nämlich nicht mehr in den mit Knochenzement gefüllten Operationshohlraum eingepresst, sondern im leeren Operationshohlraum in einer bestimmten Lage fixiert, ehe der Knochenzement eingebracht wird. Das Einbringen des Knochenzementes erfolgt dabei häufig über die Schafthöhe von distal nach proximal fortschreitend. Einen Nachteil bilden dabei die langen Fliesswege des Knochenzements entlang dem angeschnittenen Knochengewebe, auf denen eine Vermischung mit Körperflüssigkeit stattfindet und die Bindung im ausgehärteten Zustand geschwächt wird. Es ist daher weiterhin eine Zementiertechnik entwickelt worden, bei der das Einpressen des Knochenzementes abschnittsweise von distal nach proximal erfolgt, wofür in jedem Abschnitt mindestens eine seitliche Bohrung durch die Kortikalis des Knochens angelegt wird.

Aufgabe der Erfindung ist es, für diese neue Zementiertechnik einen Verankerungsschaft zu schaffen, der auf seiner ganzen Länge im Operationshohlraum in einer vorbestimmten Stellung gehalten und stabilisiert werden kann und eine abschnittsweise Füllung des Operationshohlraumes mit Knochenzement ermöglicht. Diese Aufgabe wird erfindungsgemäss dadurch gelöst, dass mehrere singuläre Elemente, den Schaft als in Umfangsrichtung geschlossene Distanzhalter umgreifend, auf der Schaftlänge in Abständen verteilt sind, wobei die Distanzhalter aus ein- oder mehrlagigen, intraoperativ verformbaren Drahtnetzen bestehen, die als nach proximal geöffnete Rinnen ausgebildet sind.

Die Distanzhalter werden beispielsweise einfach auf den Schaft aufgeschoben und auf ihm durch Klemmen gehalten oder aber gegebenenfalls auch, z.B. durch Schweissen, an den Schaft fixiert; sie gewährleisten, dass der Schaft während des abschnittsweisen Einbringens und bis zum Aushärten des Knochenzementes eine gewünschte Lage relativ zur Wand des Operationshohlraumes über seine ganze Höhe beibehält. Diese Lage ist durch die an den verschiedenen Stellen des Hohlraumes erforderlichen Dicken des Knochenzementbettes bestimmt.

Um diese Dicken in unterschiedlichen Wandbereichen verschieden machen zu können, ist es vorteilhaft, Distanzhalter unterschiedlicher Distanzbreiten auf unterschiedlichen Höhen des Schaftes anzuordnen und/oder in Unfamgsrichtung in ihrer Distanzbreite variierende Distanzhalter vorzusehen.

Im folgenden wird die Erfindung anhand eines Ausführungsbeispiels im Zusammenhang mit der Zeichnung näher erläutert.
- Fig. 1: ist ein Längsschnitt durch den proximalen Bereich eines Femurknochens, in den der neue Schaft implantiert ist;
- Fig. 2 und 3: sind die Schnitte II-II und III-III von Fig. 1.

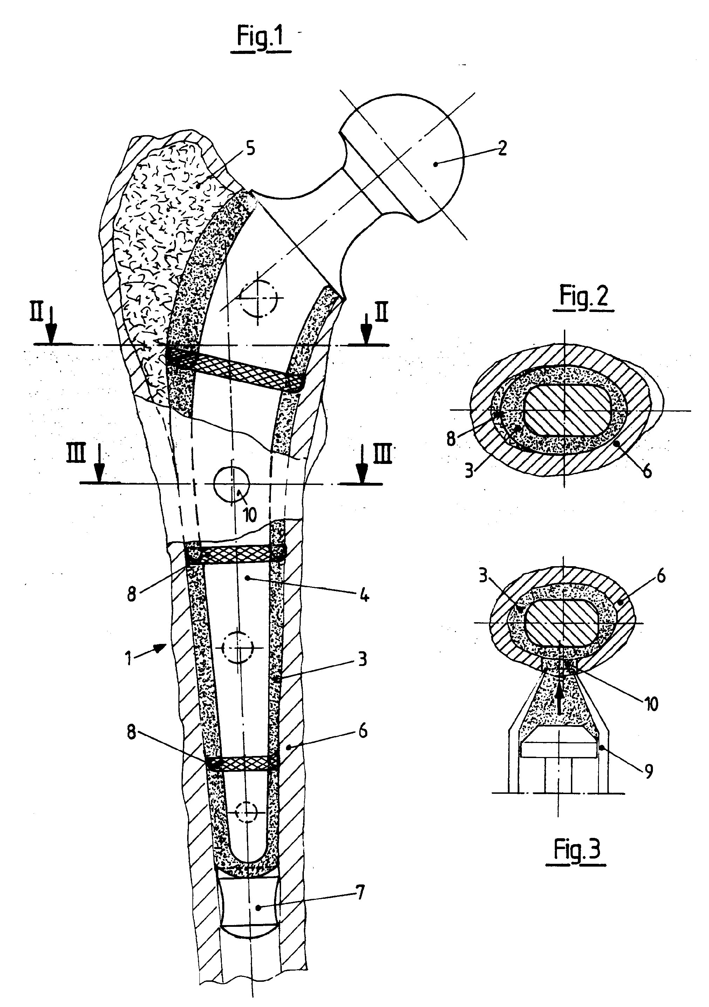

In einen Femur 1 (Fig. 1) ist eine Femurkopf-Prothese 2 eingesetzt und in einem Knochenzementbett 3 fixiert. Dieses und der Schaft 4 der Prothese 2 befinden sich im Femur 1 in einem Operationshohlraum, der durch Ausräumen der Spongiosa 5 bis zum Rand der harten Kortikalis 6 geschaffen worden ist. Nach distal ist der Operationshohlraum durch eine Markraumsperre 7 abgeschlossen.

Wie bereits erwähnt, muss bei Anwendung der neuen Zementiertechnik der Schaft 4 im ausgeräumten leeren Femur 1 stabilisiert werden, ehe der Knochenzement eingebracht werden kann. Für diese Stabilisierung ist der Schaft 4 mit von distal nach proximal auf verschiedenen Höhen verteilten Distanzhaltern 8 versehen, die an dem Schaft 4, beispielsweise durch Klemmen oder durch Schweissen, auf einer vorbestimmten Höhe fixiert sind. Die Distanzhalter 8 bestehen aus geflochtenen oder gewobenen Metalldrahtnetzen, die als nach proximal offenen Rinnen ausgeführt und in Umfangsrichtung geschlossen sind.

Wie ein Vergleich der einzelnen Distanzhalter 8 zeigt, ist die Breite sowohl zwischen einzelnen Netzen als auch längs des Umfangs eines einzelnen Distanzhalters 8 unterschiedlich, um über die Höhe und/oder den Umfang unterschiedliche Dicken des Knochenzementbettes 3 zu erhalten.

Für das Einbringen des Knochenzementes mit Hilfe einer Zementspritze 9 (Fig. 3) ist im Operationshohlraum jeder Bereich, der durch einen Distanzhalter 8 gegen die übrige Bereiche abgegrenzt ist, mit einer Bohrung 10 durch die Kortikalis 6 versehen, wie besonders in Fig. 3 verdeutlicht.

## Patentansprüche

1. Verankerungsschaft (4) zur Verankerung einer Endoprothese (2) in einem Röhrenknochen (1) mit Hilfe eines Knochenzementbettes (3) wobei der Schaft (4) durch mindestens ein auf ihn aufgeschobenes, elastisch federndes Element (8) relativ zur Wand des Operationshohlraumes in einer vorgegebenen Lage gehalten ist, dadurch gekennzeichnet, dass mehrere singuläre Elemente, den Schaft (4) als in Umfangsrichtung geschlossene Distanzhalter (8) umgreifend, auf der Schaftlänge in Abständen verteilt sind, wobei die Distanzhalter (8) aus ein- oder mehrlagigen intraoperativ verformbaren Drahtnetzen bestehen, die als nach proximal geöffnete Rinnen ausgebildet sind.

2. Verankerungsschaft nach Anspruch 1, dadurch gekennzeichnet, dass auf verschiedenen Höhen des Schaftes (4) angeordnete Distanzhalter (8) unterschiedliche Distanzbreiten haben.

3. Verankerungsschaft nach Anspruch 1 oder 2, dadurch gekennzeichnet, dass die Distanzbreite eines Distanzhalters (8) in Umfangsrichtung variiert.

## Claims

1. A stem (4) for anchoring an endoprosthesis (2) in a hollow bone (1), using a bone cement bed (3), at least one resilient element (8) being slid over the stem (4) so as to hold it in a predetermined position relative to the wall of the surgical cavity, characterised in that a number of individual elements in the form of spacers (8) which are continuous in the peripheral direction and surround the stem (4), are distributed at intervals along the stem, the spacers (8) comprising single-layer or multi-layer wire meshes which can be shaped by surgery and are in the form of channels open in the proximal direction.

2. A stem according to claim 1, characterised in that spacers (8) having varying widths are disposed at various heights along the stem (4).

3. A stem according to claim 1 or 2, characterised in that the width of a spacer (8) varies in the peripheral direction.

## Revendications

1. Tige (4) d'ancrage d'une endoprothèse (2) dans un os tubulaire (1) au moyen d'un lit (3) de ciment à os, la tige (4) étant maintenue à une position prédéterminée par rapport à la paroi de la cavité créée par opération par au moins un élément (8) capable de céder élastiquement et enfilé sur elle, caractérisée en ce que plusieurs éléments individuels formant des entretoises (8) fermées dans la direction de la périphérie et entourant la tige (4) sont répartis à distance les uns des autres sur la longueur de cette dernière, les entretoises (8) étant constituées de toiles métalliques en une ou plusieurs couches, qui sont déformables dans la cavité opératoire et qui sont conformées en goulottes ouvertes vers le côté proximal.

2. Tige d'ancrage selon la revendication 1, caractérisée en ce que les entretoises (8) disposées à différentes hauteurs de la tige (4) ont des largeurs différentes de maintien à distance.

3. Tige d'ancrage selon la revendication 1 ou 2, caractérisée en ce que la largeur de maintien à distance d'une entretoise (8) varie dans la direction de la périphérie.
